# EUROPEAN PATENT SPECIFICATION

(11) **EP 2 361 084 B1**
(45) Date of publication and mention of the grant of the patent: **01.03.2017**
(21) Application number: 09794858.2
(22) Date of filing: 20.05.2009
(51) Int. Cl.: A61K 9/70, A61K 31/14, A61K 47/00, A61P 31/00

(54) **ELECTROSTATICALLY CHARGED MULTI-ACTING NASAL APPLICATION, PRODUCT, AND METHOD**
ELEKTROSTATISCH GELADENE PRODUKTE MIT MEHRWACHWIRKUNG ZUR NASALEN ANWENDUNG UND VERFAHREN
APPLICATION NASALE POLYVALENTE CHARGÉE ÉLECTROSTATIQUEMENT, ET PRODUIT ET MÉTHODE ASSOCIÉS

(30) Priority: 07.07.2008 US 78478 P; 16.05.2009 US 467271; 03.08.2008 US 85855 P
(43) Date of publication of application: 31.08.2011
(73) Proprietor: Trutek Corp., Basking, Ridge, NJ 07920 (US)
(72) Inventor: WAHI, Ashok, L., Basking Ridge NJ 07920 (US)
(74) Representative: O'Neill, Michelle
(86) International application number: PCT/US2009/044755
(87) International publication number: WO 2010/005637

(56) References cited:
- WO-A1-2006/128981
- US-A1- 2002 006 961
- US-A1- 2003 161 790
- US-A1- 2004 071 757
- US-A1- 2006 163 149
- US-A1- 2007 243 237
- US-B1- 6 531 142
- DATABASE EMBASE [Online] ELSEVIER SCIENCE PUBLISHERS, AMSTERDAM, NL; February 2004 (2004-02), JIA Y -R ET AL: "In vitro study on the effects of hypericin and lysine hydrochloride against herpes virus", XP002666868, Database accession no. EMB-2004217223 & CHINESE JOURNAL OF CLINICAL REHABILITATION 200402 CN, vol. 8, no. 5, February 2004 (2004-02), pages 996-997, ISSN: 1671-5926

## Description

### CROSS REFERENCE TO RELATED APPLICATIONS

a) The Present Application is the non-provisional counterpart of my pending US Provisional Patent Application Serial No. 61/085,555 (the '555 Application) filed on August 3, 2008. The Present Application claims the benefit of and priority to said '555 Application.
b) The Present Application is also the non-provisional counterpart of my pending US Provisional Patent Application Serial No. 61/078,478 (the '478 Application) filed on July 7, 2008. The Present Application claims the benefit of and priority to said '478 Application.

### FIELD OF THE INVENTION

The Present Disclosure relates to the field of protective compositions against assault by various irritants and noxious substances as well as against assault by assorted microorganisms that typically gain entry into the body through the airway and/or nasal mucosa. The Present Disclosure also relates to anti-viral, anti-bacterial, and anti-microbal products and methods that involve the use of products heretofore developed for restricting the flow of airborne contaminants into the nasal passages by creating an electrostatic field in an area near about the nasal passages. This reduced the inflow of airborne contaminants to the nasal passages by capturing the contaminants and keeping them from entering the body. In the present disclosure, these electrostatically charged nasal application products capture and hold the contaminants including viruses, bacteria, and other harmful microorganisms or toxic particulates, inactivate them dermally outside the body and render them harmless.

### BACKGROUND OF THE INVENTION

The nasal passages and nasal mucosa serve as body entry points for a wide variety of noxious and toxic substances. The body's immune system responds with certain relatively harmless irritants to the nasal passages and airways with reflex responses such as coughing and sneezing. This merely re-introduces the irritants into the environment. However, when the irritant comprises microorganisms, especially those that reproduce within the body and that are transmitted by coughing and sneezing, others may become infected. When a person feels a cough or a sneeze coming on, he merely covers his nose and mouth. However, if that person is contagious, this action does little to prevent others from also becoming infected. Furthermore, the use of a tissue or handkerchief for this purpose is extremely inefficient. This limits the protection of an individual from becoming infected or infecting others.

Other means of dealing with preventing inhalation of harmful or irritating substances or of infections agents include wearing facemasks to filter out these irritants. An example of this is the simple dust mask, typically found in the hardware store or medical supply store. However, even these are inadequate and inefficient. In many localities, during flu season, one can see a large number of people wearing these dust masks in public places. The dust masks are now known to be ineffective. Another example of this preventative method is the gas mask, which is more efficient than the dust mask. Yet, even gas masks are not highly efficient with respect to microscopic and sub-microscopic microorganisms. Furthermore, they are extremely cumbersome and cannot generally be used during normal day-today activities.

Patents such as US 6,844,005 describe electrostatically charged compositions that may be applied externally in the vicinity of the nostril and attract oppositely charged materials that would otherwise be inhaled. However, those compositions simply create an electrostatic field that helps to filter out oppositely charged materials. While this action may offer suitable protection against particles that are inhaled passively, they suffer from the fact that they cannot completely deal with particulates that have their own internal means of overcoming the electrostatic forces, such as microorganisms that are motile within the air stream. Furthermore, actions by the person having those electrostatic compositions in the vicinity of the nostrils can sufficiently displace the offending particles or organisms, especially in such instances as blowing or wiping the nose, so that particles that were held captive by the former compositions could become dislodged, again set free, and be inhaled.

### OBJECTS OF THE INVENTION

- It is therefore an object of the invention to provide a formulation for electrostatically preventing harmful particulate matter from infecting an individual through nasal inhalation wherein the formulation is applied to skin or tissue of nasal passages in a thin film, said formulation comprising at least one cationic agent and at least one biocidic agent, and wherein said formulation, once applied:
   a) electrostatically attracts the particulate matter to the thin film;
   b) holds the particulate matter in place; and,
   c) kills or inactivates the particulate matter and renders said particulate matter harmless;
   wherein the at least one cationic agent is Benzalkonium Chloride and the at least one biocidic agent is Lysine HCL.
- It is another object of the disclosure to provide a composition that can be readily applied to the exterior region around the nostril and/or slightly inside the edge of the nostril or near the vicinity of the source of release with method and compositions capable of capturing particulates and microorganisms.
- It is another object of the disclosure to have the capability to hold it for a duration from being dislodged in to the air stream again.
- It is a further object of the disclosure to provide a composition that can be applied near the vicinity of the source of release or to the area around the exterior of and/or slightly inside the edge of the nostril that will inactivate, kill, or render harmless a microorganism, which has been captured and held by the composition.
- It is yet another object of the disclosure to provide a composition that can be applied to a filter substrate for improving the substrates ability to trap and hold particulates and microorganisms and to simultaneously inactivate, kill, or render harmless the microorganisms so trapped. Such filter substrate could be placed in the close proximity of the skin near the path of inhalation, near the source of release of such particulates while the inhaler is at a distance or both.
- It is still another object of the disclosure to provide a method of prophylactically preventing or of substantially reducing the risk of infection by an infectious agent without the utilization of ingested antiviral and/or antibacterial agents.
- Yet other objects of the invention will be apparent to those of ordinary skill once having benefit of the instant disclosure. In all of the foregoing objects, the deficiencies of the previously mentioned prior art are overcome by the teachings of this disclosure.

### SUMMARY OF THE INVENTION

These and other objects of the disclosure are unexpectedly achieved by an electrostatically charged composition having at least one polymeric quaternary compound in an aqueous or non-aqueous based formulation, which when applied to a surface, creates an electrostatic field such that oppositely charged airborne particulates (including microorganisms) in the vicinity of the surface are electrostatically trapped, held thereto and one or more of the microorganisms so captured is neutralized, killed, inactivated, and rendered harmless.

### DETAILED DESCRIPTION OF THE INVENTION

The present disclosure relates to anti-microorganism, anti-viral/anti-bacterial products and methods that involve the use of products that restrict the flow of airborne contaminants into the nasal passages by creating an electrostatic field in an area near about the nasal passages. Additionally, in the present disclosure, these electrostatically charged nasal application products are used to hold the contaminants including microorganisms, viruses, bacteria, and other harmful or toxic particulate outside the body and render them harmless.

Emergences of Anthrax lead to the concept of avoidance of inhaling airborne microscopic and sub-microscopic contaminants. It is the intention of the Present Disclosure to filter and render harmless materials such as anthrax spores, human corona virus, smallpox virus, influenza virus, avian flu virus, swine flu virus, rhino virus, and other biological or chemical elements/toxins/irritants, and the like, prior to their entering the nasal passages.

Airborne microorganisms are a major cause of respiratory ailments in humans, causing allergies, asthma, and pathogenic infections of the respiratory tract. Airborne fungal spores are also important agents that spread diseases. Respiratory diseases cause many fatalities and are a cause of great concern. During a sneeze, millions of tiny droplets of water and mucus are expelled at a high velocity. The droplets contain viral particles and/or bacteria. This is a means of transmission of several diseases by inhaled airborne particles as follows:

| **VIRAL DISEASES (virus type in brackets)** | **BACTERIAL DISEASES (bacterial name in brackets)** |
|---|---|
| Chickenpox (Varicella) | Whooping cough (*Bordetella pertussis*) |
| Flu (Influenza) | Meningitis (*Neisseria* species) |
| Measles (Rubeola) | Diphtheria *(Corynebacterium diphtheriae*) |
| German measles (Rubella) | Pneumonia (*Mycoplasma pneumoniae, Streptococcus* species) |
| Mumps (Mumps) | Tuberculosis (*Mycobacterium tuberculosis*) |
| Smallpox (Variola) | |
| SARS (Human Corona) | Anthrax (Anthracsis bacterium) |

Diseases caused by environmental particulates include, but are not limited to the following:

| **ENVIRONMENTAL PARTICULATE DISEASES** | **SOURCE** |
|---|---|
| Psittacosis (*Chlamydia psittaci*) | Dried, powdery droppings from infected birds (parrots, pigeons, etc.) |
| Legionnaire's disease (*Legionella pneumophila*) | Droplets from air-conditioning systems, water storage tanks, etc., where the bacterium grows. |
| Acute allergic alveolitis (various fungal and actinomycete spores) | Fungal or actinomycete spores from decomposing organic matter (composts, grain stores, hay, etc.) |
| Aspergillosis (*Aspergillus fumigatus, A. flavus, A. niger)* | Fungal spores inhaled from decomposing organic matter. |
| Histoplasmosis (*Histoplasma capsulatum*) | Spores of the fungus, in old, weathered bat or bird droppings. |
| Coccidioidomycosis (*Coccidioides immitis*) | Spores in air-blown dust in desert regions (Central, South and North America) where the fungus grows in the soil. |

To accomplish the present disclosure, a formulation having at least one polyquaternary ammonium compound is prepared, such compounds, alone or together capable of creating an electrostatic field on and around a surface to which it is applied, including surfaces such as skin, textile (woven and non-woven), and hard surfaces, such as floors, walls, wood, metal, plastic, etc. The formulation is generally aqueous based, but may include non-aqueous solvents used which are compatible with the other formulation components and the application surface to which it is applied. Preferably, the formulation is an aqueous formulation. In addition to the polyquaternary ammonium compound, the composition includes at least. Furthermore, the composition may contain, but is not required to contain various thickeners, gellants, fragrances, colorants, emollients, humectants, and generally other suitable components that are compatible with the end use application and the other components of the formulations. Thus, a composition of the disclosure that is intended to be applied to a filter substrate that is perhaps used as a mask with an additional liner between a user and the filter substrate may utilize materials that would not be compatible with direct contact with skin, although it is preferable that all of the components are compatible with direct application to the skin as a means of limiting reaction due to inadvertent contact between the composition and the skin.

A formulation of the disclosure comprises:
- water,
- at least one quaternary thickener,
- a preservative,
- a conditioner,
- an emulsifier,
- a biocidic agent, and
- a neutralizing agent added to adjust and achieve a pH in the range of 5.0 to 6.8.

It may further comprise without limitation a combination of the following:
- a surfactant,
- a thickener,
- an emollient,
- a humectant, and
- a binder.

In an exemplary example of such a formulation, a quaternary thickener may comprise without limitation, at least one of the following:
- Polyquaternium - 10
- Polyquaternium - 22
- Polyquaternium - 67
- Polyquaternium - 70
- Polyquaternium - 72
- Polyquaternium - 88
- Cocodimonium Hydroxypropyl Hydrolyzed Keratin
- Hydroxypropyltrimonium Wheat Protein

Benzalkonium Chloride may also serve the same function, but it is also a cationic agent as well as a biocide. Another biocide that may be used is Lysine HCL.

In an exemplary example of such a formulation, an emulsifier may comprise without limitation, at least one of the following:
- Cetyl Alcohol (which can also serve as a thickener)
- Cetearyl Alcohol
- Glyceryl Stearate
- Ceteareth -20
- PEG - 40 Stearate
- Dicetyl Phosphate
- Ceteth - 10 Phosphate

In an exemplary example of such a formulation, the emollient may be Isocetyl Behenate without limitation. The thickener may be Cetyl Alcohol or Stearyl Alcohol without limitation.

In an exemplary example of such a formulation, a preservative may comprise without limitation, at least one of the following:
- Phenoxyethanol;
- Methylparaben;
- Butylparaben;
- Ethylparaben;
- Propylparaben;
- Isobutylparaben.

Examples of typical formulations found to be effective appear in the ten tables that follow. Percentages are given by weight.

**TABLE 1**

| **Ingredient** | **Percent Range** | **Function** |
|---|---|---|
| Water | 62% - 80% | Solvent, Moisturizer |
| Gluconolactone, Sodium Benzoate | 1% | Preservative |
| Lysine HCL | 1% | Conditioner |
| Polyquaternium - 67 | 3% - 6% | Conditioner |
| Octoxynol - 9 | 2% - 5% | Surfactant |
| Polyquaternium - 72 | 6% - 10% | Conditioner |
| Polyquaternium - 70 Dipropylene Glycol | 0.5% - 1% | Conditioner |
| Isocetyl Behenate | 4% - 6% | Emollient |
| Stearyl Alcohol | 1% - 3% | Thickener |
| Cetyl Alcohol | 0.25% - 1% | Thickener |
| Ceteareth -20, PEG - 40 Stearate, Cetearyl Alcohol | 1% - 2% | Emulsifier |
| Water, Hydrolyzed Algin | 0.5% - 1.5% | Conditioner |
| Hydrolized Soy Protein | 0.25% - 1% | Conditioner |

**TABLE 2**

| **Ingredient** | **Percent Range** | **Function** |
|---|---|---|
| Water | 72% - 88% | Solvent, Moisturizer |
| Phenoxyethanol | 1% | Preservative |
| Methylparaben, Propylparaben, Butylparaben, Ethylparaben, Isobutylparaben | | |
| Lysine HCL | 1% | Conditioner, Biocide |
| Polyquaternium - 67 | 3% - 6% | Conditioner, Quaternary |
| Nonoxynol - 10 | 2% - 4% | Surfactant |
| Cocodimonium | 0.5% - 2% | Conditioner, Quaternary |
| Hydroxypropyl | | |
| Hydrolyzed Keratin | | |
| Polyquaternium - 72 | 0.5% - 2% | Conditioner, Quaternary |
| Polyquaternium - 88 | 1% - 4% | Conditioner, Quaternary |
| Cetearyl Alcohol, Glyceryl Stearate | 1% - 4% | Emulsifier |
| Emulsifier, PEG - 40 Stearate, Ceteareth - 20 | | |
| Cetearyl Alcohol, Dicetyl Phosphate, Ceteth - 10 Phosphate | 0.5% | Emulsifier |
| Benzalkonium Chloride | 0.25% - 1% | Cationic, Quaternary, Biocide |
| Hydroxypropyltrimonium Wheat Protein | 1% | Conditioner, Quaternary |
| Sodium Hydroxide | 0.01% - 0.05% | Neutralizing Agent |

**TABLE 3**

| **Ingredient** | **Percent Range** | **Function** |
|---|---|---|
| Water | 67% - 87% | Solvent, Moisturizer |
| Phenoxyethanol, Methylparaben, Propylparaben, Butylparaben, Ethylparaben, Isobutylparaben | 1% | Preservative |
| Lysine HCL | 1% | Conditioner, Biocide |
| Polyquaternium - 67 | 3% - 7% | Conditioner, Quaternary |
| Polyquaternium - 72 | 3% - 7% | Conditioner, Quaternary |
| Cocodimonium Hydroxypropyl Hydrolized Keratin | 1% - 4% | Conditioner, Quaternary |
| Polyquaternium - 88 | 1% - 4% | Conditioner, Quaternary |
| Cetyl Alcohol | 1.5% - 2.5% | Thickener |
| Cetearyl Alcohol, Glyceryl PEG - 40 | 1% - 4% | Emulsifier |
| Stearate, Ceteareth - 20 | | |
| Benzalkonium Chloride | 0.25% - 1% | Cationic, Quaternary, Biocide |
| Hydroxypropyltrimonium Wheat Protein | 1% | Conditioner, Quaternary |
| Sodium Hydroxide | .025% - .075% | Neutralizing Agent |

**TABLE 4**

| **Ingredient** | **Percent Range** | **Function** |
|---|---|---|
| Water | 71% - 83% | Solvent, Moisturizer |
| Phenoxyethanol, Methylparaben, Propylparaben, Butylparaben, Ethylparaben, Isobutylparaben | 1% | Preservative |
| Lysine HCL | 1% | Conditioner, Biocide |
| Polyquaternium - 67 | 4% - 6% | Conditioner, Quaternary |
| Polyquaternium - 72 | 4% - 6% | Conditioner, Quaternary |
| Cocodimonium Hydroxypropyl Hydrolyzed Keratin | 2% - 4% | Conditioner, Quaternary |
| Polyquaternum - 88 | 1% - 3% | Conditioner, Quaternary |
| Cetyl Alcohol | 2% | Thickener |
| Cetearyl Alcohol, Glyceryl Stearate, PEG - 40 Stearate, Ceteareth - 20 | 1% - 3.5% | Emulsifier |
| Benzalkonium Chloride | 0.25% - 1% | Cationic, Quaternary, Biocide |
| Hydroxypropyltrimonium Wheat Protein | 1% | Conditioner, Quaternary |
| Sodium Hydroxide | .025% - .075% | Neutralizing Agent |

**TABLE 5**

| **Ingredient** | **Percent Range** | **Function** |
|---|---|---|
| Water | 73% - 85% | Solvent, Moisturizer |
| Phenoxyethanol, Methylparaben, Propylparaben, Butylparaben, Ethylparaben, Isobutylparaben | 1% | Preservative |
| Lysine HCL | 1% | Conditioner, Biocide |
| Polyquaternium - 67 | 2% - 3% | Conditioner, Quaternary |
| Polyquaternium - 72 | 4% - 6% | Conditioner, Quaternary |
| Cocodimonium Hydroxypropyl Hydrolyzed Keratin | 2% - 4% | Conditioner, Quaternary |
| Polyquaternium - 88 | 1% - 3% | Conditioner, Quaternary |
| Cetyl Alcohol | 2% | Thickener |
| Cetearyl Alcohol, Glyceryl Stearate, PEG - 40 Stearate, Ceteareth - 20 | 1% - 3% | Emulsifier |
| Benzalkonium Chloride | 0.25% - 1% | Cationic, Quaternary, Biocide |
| Hydroxypropyltrimonium Wheat Protein | 1% | Conditioner, Quaternary |
| Sodium Hydroxide | 0.05% - 0.75% | Neutralizing Agent |

**TABLE 6**

| **Ingredient** | **Percent Range** | **Function** |
|---|---|---|
| Water | 69% - 85% | Solvent, Moisturizer |
| Phenoxyethanol, Methylparaben, Propylparaben, Butylparaben, Ethylparaben, Isobutylparaben, | 1% | Preservative |
| Lysine HCL | 1% | Conditioner, Biocide |
| Polyquaternium - 10 | 0.25% - 0.85% | Conditioner, Quaternary |
| Polyquaternium - 67 | 1.5% - 3.5% | Conditioner, Quaternary |
| Polyquaternium - 72 | 4% - 6% | Conditioner, Quaternary |
| Cetyl Alcohol | 1% - 3% | Thickener |
| Cocodimonium Hydroxypropyl Hydrolyzed Keratin | 2% - 4% | Conditioner, Quaternary |
| Polyquaternium - 88 | 1% - 3% | Conditioner, Quaternary |
| Polyquaternium - 22 | 1% - 3% | Conditioner, Quaternary |
| Cetearyl Alcohol, Glyceryl Stearate, PEG - 40 Stearate, Ceteareth - 20 | 1% - 3% | Emulsifier |
| Benzalkonium Chloride | 0.25% - 1% | Conditioner, Quaternary, Biocide |
| Hydroxypropyltrimonium Wheat Protein | 1% | Conditioner, Quaternary |
| Sodium Hydroxide | 0.05% - 0.75% | Neutralizing Agent |

**TABLE 7**

| **Ingredient** | **Percent Range** | **Function** |
|---|---|---|
| Water | 67% - 86% | Solvent, Moisturizer |
| Phenoxyethanol, Methylparaben, Butylparaben, Ethylparaben, Isobutylparaben | 1% | Preservative |
| Lysine HCL | 1% | Conditioner, Biocide |
| Polyquaternium - 10 | 1% - 4% | Conditioner, Quaternary |
| Polyquaternium - 67 | 1% - 4% | Conditioner, Quaternary |
| Polyquaternium - 72 | 0.5% - 1.5% | Conditioner, Quaternary |
| Cocodimonium Hydroxypropyl Hydrolized Keratin | 0.5% - 1.5% | Conditioner, Quaternary |
| Microcare Quat CTC 30 | 1% - 3% | Conditioner, Quaternary |
| Polyquaternium - 88 | 1% - 3% | Conditioner, Quaternary |
| Polyquaternium - 22 | 1% - 3% | Conditioner, Quaternary |
| Cetyl Alcohol | 3% - 5% | Thickener |
| Cetearyl Alcohol, Glyceryl Stearate, PEG - 40 Stearate, Ceteareth - 20 | 2% - 3% | Emulsifier |
| Benzalkonium Chloride | 0.25% - 1% | Conditioner, Quaternary, Biocide |
| Hydroxypropyltrimonium Wheat Protein | 1% | Conditioner, Quaternary |
| Sodium Hydroxide | 0.05% - 0.1% | Neutralizing Agent |

**TABLE 8**

| **Ingredient** | **Percent Range** | **Function** |
|---|---|---|
| Water | 58% - 74% | Solvent, Moisturizer |
| Phenoxyethanol, Methylparaben, Propylparaben, Butylparaben, Ethylparaben, Isobutylparaben | 1% | Preservative |
| Lysine HCL | 1% | Conditioner, Biocide |
| Glycerin | 10% | Humectant |
| Glyceryl Acetate/Acrylic Acid Copolymer | 1% | Conditioner, Humectant |
| Polyquaternium - 10 | 1% - 4% | Conditioner, Quaternary |
| Polyquaternium - 67 | 1% - 3% | Conditioner, Quaternary |
| Polyquaternium - 72 | 0.5% - 1.5% | Conditioner, Quaternary |
| Cocodimonium Hydroxypropyl Hydrolyzed Keratin | 0.5% - 1.5% | Conditioner, Quaternary |
| Cetrimonium Chloride | 1% - 3% | Conditioner, Quaternary |
| Polyquaternium - 88 | 1% - 3% | Conditioner, Quaternary |
| Polyquaternium - 22 | 1% - 3% | Conditioner, Quaternary |
| Cetyl Alcohol | 4% | Thickener |
| Cetearyl Alcohol, Glyceryl Stearate, PEG - 40 Stearate, Ceteareth - 20 | 2% - 3% | Emulsifier |
| Polybutene | 4% | Binder |
| Benzalkonium Chloride | 0.25% - 1% | Conditioner, Quaternary, Biocide |
| Hydroxypropyltrimonium Wheat Protein | 1% | Conditioner, Quaternary |
| Sodium Hydroxide | .0.05% - 0.1% | Neutralizing Agent |

**TABLE 9**

| **Ingredient** | **Percent Range** | **Function** |
|---|---|---|
| Water | 54% - 73% | Solvent, Moisturizer |
| Phenoxyethanol, Methylparaben, Propylparaben, Butylparaben, Ethylparaben, Isobutylparaben | 1% | Preservative |
| Lysine HCL | 1% | Conditioner, Biocide |
| Glycerin | 8% | Humectant |
| Glyceryl Acetate/Acrylic Acid Copolymer | 1% | Conditioner, Humectant |
| Polyquaternium - 10 | 1% - 4% | Conditioner, Quaternary |
| Polyquaternium - 67 | 1% - 4% | Conditioner, Quaternary |
| Polyquaternium - 72 | 0.5% - 2% | Conditioner, Quaternary |
| Cocodimonium Hydroxypropyl Hydrolyzed Keratin | 0.5% - 2% | Conditioner, Quaternary |
| Cetrimonium Chloride | 1% - 3% | Conditioner, Quaternary |
| Polyquaternium - 88 | 1% - 3% | Conditioner, Quaternary |
| Polyquaternium - 22 | 1% - 3% | Conditioner, Quaternary |
| Cetyl Alcohol | 4% | Thickener |
| Cetearyl Alcohol, Glyceryl Stearate, PEG - 40 Stearate, Ceteareth - 20 | 2% - 3% | Emulsifier |
| Polybutene | 3% - 4% | Binder |
| Benzalkonium Chloride | 0.25% - 1% | Conditioner, Quaternary, Biocide |
| Hydroxypropyltrimonium Wheat Protein | 1% | Conditioner, Quaternary |
| Sodium Hydroxide | 0.05% - 0.1% | Neutralizing Agent |

**TABLE 10**

| **Ingredient** | **Percent Range** | **Function** |
|---|---|---|
| Water | 52% - 71% | Solvent, Moisturizer |
| Phenoxyethanol, Methylparaben, Propylparaben, Butylparaben, Ethylparaben, Isobutylparaben | 1% | Preservative |
| Lysine HCL | 1% | Conditioner, Biocide |
| Glycerin | 9% | Humectant |
| Glyceryl Acetate/Acrylic Acid Copolymer | 1% | Conditioner, Humectant |
| Polyquaternium - 10 | 1% - 3.5% | Conditioner, Quaternary |
| Polyquaternium - 67 | 1% - 3% | Conditioner, Quaternary |
| Polyquaternium - 72 | 0.5% - 2% | Conditioner, Quaternary |
| Cocodimonium Hydroxypropyl Hydrolyzed Keratin | 0.5% - 2% | Conditioner, Quaternary |
| Cetrimonium Chloride | 1% - 3% | Conditioner, Quaternary |
| Polyquaternium - 88 | 1% - 3% | Conditioner, Quaternary |
| Polyquaternium - 22 | 1% - 3% | Conditioner, Quaternary |
| Cetyl Alcohol | 4% | Thickener |
| Cetearyl Alcohol, Glyceryl Stearate, PEG - 40 Stearate, Ceteareth - 20 | 1% - 4% | Emulsifier |
| Polybutene | 5% - 6% | Binder |
| Benzalkonium Chloride | 0.25% - 1% | Conditioner, Quaternary, Biocide |
| Hydroxypropyltrimonium Wheat Protein | 1% | Conditioner, Quaternary |
| Sodium Hydroxide | 0.05% - 0.1% | Neutralizing Agent |

All of the formulations described in TABLE 1-10 representing various examples of the Present Disclosure operate in the manner that was disclosed herein. The same results may be achieved by varying the percentages for the active and inactive ingredients. Varying the percentages for the active ingredients affects the potency of the formulation. Varying the percentages for the inactive ingredients affects the consistency of the formulation. The desired results may be achieved by varying the ingredients and their amounts by those skilled in the art without undue experimentation.

## Claims

1. A formulation for electrostatically preventing harmful particulate matter from infecting an individual through nasal inhalation wherein the formulation is applied to skin or tissue of nasal passages in a thin film, said formulation comprising at least one cationic agent and at least one biocidic agent, and wherein said formulation, once applied:
a) electrostatically attracts the particulate matter to the thin film;
b) holds the particulate matter in place; and,
c) kills or inactivates the particulate matter and renders said particulate matter harmless;
wherein the at least one cationic agent is Benzalkonium Chloride and the at least one biocidic agent is Lysine HCL.

## Patentansprüche

1. Formulierung zur elektrostatischen Verhinderung, dass schädliche Partikelmasse ein Individuum durch nasale Inhalation infiziert, wobei die Formulierung in einer dünnen Schicht auf Haut oder Gewebe von Nasengängen aufgetragen wird, wobei die Formulierung mindestens ein kationisches Mittel und mindestens ein biozides Mittel umfasst und wobei die Formulierung, sobald aufgetragen:
a) die Partikelmasse elektrostatisch an die dünne Schicht anzieht;
b) die Partikelmasse an Ort und Stelle hält; und
c) die Partikelmasse abtötet oder deaktiviert und die Partikelmasse harmlos macht;
wobei das mindestens eine kationische Mittel Benzalkoniumchlorid ist und das mindestens eine biozide Mittel Lysin HCL ist.

## Revendications

1. Formulation permettant d'empêcher, par effet électrostatique, qu'une matière particulaire nocive n'infecte un individu par inhalation nasale, dans laquelle la formulation s'applique sur de la peau ou sur du tissu des voies nasales sous forme de film mince, ladite formulation comprenant au moins un agent cationique et au moins un agent biocide, et dans laquelle ladite formulation, lorsqu'elle a été appliquée :
a) attire la matière particulaire vers le film mince par effet électrostatique ;
b) maintient la matière particulaire en place ; et
c) tue ou inactive la matière particulaire et rend inoffensive ladite matière particulaire ;
dans laquelle l'au moins un agent cationique est du chlorure de benzalkonium et l'au moins un agent biocide est du chlorhydrate de lysine.
